# EUROPEAN PATENT SPECIFICATION

(11) **EP 4 520 245 B1**
(45) Date of publication and mention of the grant of the patent: **28.10.2026**
(21) Application number: 23195488.4
(22) Date of filing: 05.09.2023
(51) Int. Cl.: A61B 1/00, A61B 1/015

(54) **ENDOSCOPE COMPRISING A SUCTION VALVE**
ENDOSKOP MIT SAUGVENTIL
ENDOSCOPE COMPRENANT UNE SOUPAPE D'ASPIRATION

(43) Date of publication of application: 12.03.2025
(73) Proprietor: Ambu A/S, 2750 Ballerup (DK)
(72) Inventor: MORTON, Alistair David, 2300 Copenhagen (DK)
(74) Representative: Winter, Brandl - Partnerschaft mbB

(56) References cited:
- DE-B4- 19 610 312
- JP-A- 2018 164 565
- JP-A- H07 349
- JP-A- H1 156 770

## Description

The present disclosure relates to an endoscope comprising a proximal endoscope handle or interface and an insertion cord extending from the endoscope handle or interface, the insertion cord being configured to be inserted into a patient's body cavity. The endoscope further comprises a suction valve mounted in the endoscope handle or interface. The present disclosure further relates to a method of assembling the suction valve of the endoscope and a system comprising an endoscope and a video processing apparatus (VPA).

### Related art

Endoscopes and similar specialized instruments such as bronchoscopes, arthroscopes, colonoscopes, laparoscopes, gastroscopes, and duodenoscopes are well-known from the related art and are typically used for visual examination and diagnosis of hollow organs and body cavities, as well as to assist in surgery, e.g. for a targeted tissue sampling. Both reusable and disposable endoscopes are known from the related art. Known endoscopes usually comprise: an endoscope handle or interface; an insertion cord extending from the endoscope handle or interface to be inserted into a patient's body cavity and comprising an insertion tube, a bending section, and a distal tip unit; and a working channel. The working channel extends from the working channel access port at the endoscope handle to the distal tip unit.

When examining an object such as a body cavity or hollow organ with an endoscope it is desirable to have a clear and good view or visibility of the examined object. However, the visibility of such an object is often affected by undesirable fluid or mucus, which may obstruct visibility of the object to be examined. Furthermore, it may be desirable to remove tissue parts or particles during the surgery for example to extract a sample for further analysis, or to provide better visibility. It is thus desirable to remove such undesirable fluid or mucus, as well as such tissue parts or particles, using a suction device, such as a vacuum pump.

It is desirable that the surgeon can start and interrupt the suction at any time. Basically, it would be possible to control a vacuum pump performance for this purpose. However, this is associated with considerable delays caused by a startup and a shutdown of the pump motor.

In order to overcome these disadvantages, suction valves have become established in endoscopes, with which the effect of the suction power constantly applied by the vacuum pump can be applied or switched off. Further general requirements for such a suction valve may be that the suction valve is easy and quick to operate, and closes reliably and safely.

Various suction valves for endoscopes are known in the related art.

Basically, suction valves comprising a housing and a piston that is movable within the housing are well-known.

For example, US 5 871 441 A discloses a suction valve that can be connected to a working channel of an endoscope and includes a preferably cylindrical housing, a piston, a spring and a button. The piston is provided with a flow channel and is movably accommodated within the housing. The button preferably on top of the piston is connected to the piston such that the piston is movable together with the button. An operator can press the button to move the piston from a valve closed state to a valve open state. The spring ensures that the valve is usually in the valve closed state, in particular when the operator does not press down the button. The housing is provided with an inlet opening that is connected to the working channel of the endoscope and with an outlet opening that is connected to a suction device. Usually, the suction device is active or running. When the valve is in the valve closed state an outer circumferential surface of the cylindrically shaped piston blocks the inlet opening. When the operator presses down the button, the piston is moved downwards such that the flow channel of the piston connects the inlet opening of the housing with the outlet opening of the housing, and a fluid flow through the suction valve is enabled. Hence, fluid or mucus or tissue parts and/ or particles can be drawn from the working channel of the endoscope through the suction valve.

US 5 299 561 A discloses a suction valve integrated into an endoscope handle. The suction valve comprises a suction button, which is pressed and released by the operator to activate or deactivate suction. When the valve is open, the volume flow flows around a stem, whereby the volume flow is diverted several times within the suction valve.

JP H06 - 70 880 A discloses a suction control device for an endoscope comprising a stem, the stem being axially movable in the valve between at least an open state and a closed state. The suction control device comprises an elastic closing film, which is pierced and opened by the stem when the valve is opened. Due to the piercing of the elastic film, edges are formed in the open state at which turbulence of the volume flow may occur. Furthermore, the suction control device has a large number of components, which makes the suction control device complex and expensive.

JP 5 552 014 B2 discloses a suction button for an endoscope, the suction button comprising a plunger, which is axially movable between at least an open state and a closed state. In the open state of the valve, the volume flow is guided through a partial recess in the plunger, whereby the volume flow is deflected at least twice in the suction button of the endoscope.

DE 196 10 312 B4 discloses a device for controlling a suction passage of an endoscope. The device includes a cylinder arranged between an insertion pipe and a suction pipe. A piston inside the cylinder can move in axial direction to connect or disconnect the insertion and suction pipes.

JP H11 56770 A discloses a suction operation part including an operation button that is pressed (moving in axial direction) to elastically downwardly deform the upper half part of a tubular body to connect an air suction hole and a communicating hole so that air flows from a small diameter part to a port part in a curve.

JP 2018 164565 A discloses a suction operation device in the operation section of an endoscope. The suction operation device has a cylinder, a valve body, and a piston that can be moved axially.

JP H07 349 A discloses a tube line selector valve for an endoscope capable of surely switching a communicated state and a closed state of tube lines.

For related art solutions it usually applies that fluid, mucus and tissue sucked-off by the suction device is sharply deflected several times within the suction valve, which considerably reduces the volume flow, increases the load on the vacuum pump, and reduces the pumping capacity and suction performance. Furthermore, narrowing of the valve increases the risk of clogging, which leads to interruptions in the treatment process and thus endangers patient safety. Furthermore, the suction valves known in the related art are complex with a large number of parts, which makes the valves expensive to manufacture, which is of considerable disadvantage especially for the use of such a suction valve with a single-use endoscope.

### Brief description of the disclosure

The object of the present disclosure is therefore to overcome or at least reduce the disadvantages of the related art. More specifically, it is the purpose of the present disclosure to provide an endoscope having a suction valve, wherein the suction valve provides an appropriate flow rate in an open state of the suction valve, reduces a risk of clogging, and is inexpensive and easy to manufacture.

The invention is defined by the features of independent claims 1 and 12. Preferred embodiments are defined in the dependent claims. Aspects, embodiments and examples disclosed herein which do not fall within the scope of the appended claims do not form part of the invention and are merely provided for illustrative purposes.

In detail, the present disclosure relates to an endoscope that comprises a proximal endoscope handle or interface, an insertion cord extending from the endoscope handle or interface and configured to be inserted into a patient's body cavity and a working channel configured to let fluid and mucus sucked from the patient's body cavity flow therethrough. The endoscope handle or interface comprises a suction valve configured to control a suction through the working channel and having a valve closed state and a valve open state. The suction valve comprises: a valve body having a valve cylinder, an inlet connected to the working channel, and an outlet provided and configured to be connected to a suction device; and a stem movable axially in the valve cylinder between at least a first position, in which the suction valve is in the valve closed state, and a second position, in which the suction valve is in the valve open state. The inlet is connected to and extends away from an outer cylindrical surface of the valve cylinder. The outlet is connected to and extends away from the outer cylindrical surface of the valve cylinder. The stem comprises a curved flow guide configured to guide the fluid and/or mucus from the inlet to the outlet in the valve open state. The inlet, the outlet and the curved flow guide are arranged and adapted to each other so as to provide a curved sideways flow path for the fluid and the mucus through the suction valve in the valve open state.
The curved sideways flow path is provided by an enclosed angle between a first orientation or center axis of the inlet and a second orientation or center axis of the outlet being larger than 100° and smaller than 180° seen in a sectional side view of the endoscope handle in an assembled state of the suction valve, in combination with the curved flow guide of the stem. The curved flow guide is configured to guide the fluid and the mucus from the inlet to the outlet in the valve open state of the suction valve.
In other words, the present disclosure relates to the endoscope, preferably a single use endoscope, comprising the endoscope handle or interface. In other words, the handle may be substituted by the interface. Alternatively, it is conceivable that the interface is formed on the handle. The handle may contain essential control elements, such as a lever for controlling bending of the endoscope, for the endoscope and ports for accessories such as a video processing apparatus or the suction device, for example in the form of a vacuum pump. The handle is preferably designed in such a way that it can be ergonomically gripped and operated by the user or operator or surgeon with just one hand. This means that all important operating elements on the handle are preferably arranged in such a way that the user can operate them with one hand without having to change a position of his/ her hand. The handle may comprise a shell. Preferably, the shell is made from a plastic material. Furthermore, the shell preferably comprises two half shells. In case of a provision of a proximal endoscope interface, said endoscope interface may be prepared or provided to be connectable to e.g. a robotic arm.
The endoscope further comprises the insertion cord, preferably having a distal tip unit formed at the distal end of the insertion cord. The distal tip unit may comprise an image capturing means like a camera and a light emitting device like a LED.
Furthermore, the endoscope comprises the working channel. The working channel is provided and configured to transport fluid, mucus and tissue parts or particles from the patient's body cavity. Furthermore, the working channel may be configured to guide a surgical tool or instrument through it to a surgical location in the patient's body. For this purpose, the working channel may have a Y-connector in the handle that allows insertion of the surgical tool or instrument into the working channel.
The endoscope further comprises the suction valve arranged in the endoscope handle or interface. The suction valve described in the present disclosure may, if applicable, also be claimed individually.
The suction valve comprises the valve body, which may also be designated as a valve housing. The valve body may be formed cylindrically at least in portions and may thus be described as including a valve cylinder that extends in an axial direction of the suction valve.
The inlet and the outlet of the suction valve are preferably formed as integral parts or portions of the valve body. The inlet and the outlet may have substantially a geometry of a pipe section, preferably of a pipe section with an annular cross-section.
The inlet and outlet extend away from an outer cylindrical surface of the valve cylinder of the valve body, preferably straight or linear. The orientation or center axis of the inlet seen in a sectional side view of the endoscope handle in an assembled state of the suction valve may extend away from the outer cylindrical surface of the valve cylinder both in a radial and in an axial direction of the valve cylinder. The orientation or center axis of the outlet seen in a sectional side view of the endoscope handle in an assembled state of the suction valve may extend away from the outer cylindrical surface of the valve cylinder both in a radial and in an axial direction. It is also conceivable that the inlet or the outlet extends away purely radially from the valve cylinder, i.e. does not extend axially in the axial direction of the valve cylinder.
The inlet has thus preferably the first center axis or orientation. The first center axis is a virtual auxiliary axis and extends from a center position of the inlet in the direction of the extension of the inlet seen in a sectional side view of the endoscope handle in the assembled state of the suction valve. The inlet is provided and configured to be connected to the working channel. The inlet may be connected directly to the working channel. Alternatively, the inlet can be connected to the working channel via a suction channel, especially preferably to the Y-connector of the working channel. The working channel may at least partly be provided by the working channel tube. The suction channel may e.g. be configured as a separate hose or tube.

The outlet has preferably the second center axis or orientation. The second center axis is a virtual auxiliary axis and extends from a center position of the outlet in the direction of the extension of the outlet seen in a sectional side view of the endoscope handle in the assembled state of the suction valve. The outlet is provided and configured to be connected to the suction device. The outlet may be connected directly to the suction device. Alternatively, a hose may be provided between the outlet and the suction device. Alternatively or additionally, at least one coupling element and/or at least one deflector element and/or at least one pipe element may be provided between the outlet and the suction device.

The suction valve further comprises the stem. The stem is arranged in the valve body in such a way that the stem is able to move in the axial direction of the suction valve in the valve body, especially in the valve cylinder of the valve body. The axial movement, at least in one direction, can be caused by pushing a button. The button may be a button portion which is preferably formed integrally with a remainder of the stem at an end portion of the stem. Alternatively, the button may be a button element attached to the stem, in particular to the end portion of the stem, which is positioned outside of the valve body at least in the closed state of the valve. The button portion or element may protrude axially from the valve body, so that a user can press the button portion or element in order to move the stem axially in the valve body. The stem can be moved in the valve body in such a way that it shifts the suction valve at least between the valve closed state of the suction valve in its first position and the valve open state of the suction valve in its second position. In other words, the stem may be configured to seal between an inlet opening and an outlet opening in the suction valve closed state, and the stem may be configured to enable a fluid flow between the inlet opening and the outlet opening of the valve body in the suction valve open state. In still other words, the suction valve may be configured to manually control the suction through the working channel, in particular by the user pressing the button towards the housing of the endoscope. In this way, the user can easily control the suction valve and can switch between the valve closed state and the valve open state.

The suction valve, in particular the parts thereof, may be made of a plastic material or polymer. Especially preferred, the suction valve, in particular parts thereof, preferably the valve body and the stem, may be manufactured in an injection molding process, i.e. may be injection molded parts.

The enclosed angle between the first center axis of the inlet and the second center axis of the outlet is larger than 100° and smaller than 180°, in order to provide - together with the curved flow guide of the stem - the curved sideways flow path. The enclosed angle is larger than 100°. Preferably, the enclosed angle is larger than 110°. Preferably, the enclosed angle is smaller than 170°. In a particularly preferred embodiment, the enclosed angle is approximately 120°.

It is to be understood that in the formulations "larger than" and "smaller than", the limit value itself is not part of the claimed range of values in each case.

Further, the stem comprises the curved flow guide. The curved flow guide is a section with a curved surface, which is part of the stem or mounted to the stem. Preferably, the curved flow guide has exactly one curved surface to guide the fluid and mucus and is free of edges and/or discontinuities. Preferably, the curved flow guide is formed integrally with the stem, i.e. is an integral part or portion of the stem.

Providing such a suction valve in an endoscope can ensure that the fluid and mucus passing through the suction valve in the valve open state of the suction valve is deflected as little as possible and thus a suitable flow through the suction valve can be achieved. Such flow advantageously reduces or even prevents blockages in the suction valve, which would disrupt a surgical procedure. Furthermore, the reduced number of components means that the assembly effort and thus the costs of the suction valve are reduced considerably, which is particularly important for a single-use endoscope. In other words, to improve the flow through the valve, the stem is provided with a curved flow guide which in the pressed or opened state of the valve, i.e. the valve open state, smoothly guides the flow through the stem and prevents the chicane like obstruction and flow of the existing suction valves known from the related art. In other words, the curved flow guide, in particular the curved surface, is designed such that it provides a curved, continuous transition between the inlet and the outlet in the valve open state.

The curved flow guide comprises at least a first, preferably infinitesimally small, surface or portion tangential to, i.e. parallel to a direction of the first center axis or orientation of the inlet and a second (infinitesimally small) surface portion tangential to, i.e. parallel to a direction of the second center axis or orientation of the outlet.

In other words, a curvature of the curved flow guide is configured such that the flow guide at a first end portion facing the inlet is oriented substantially in the direction or a direction parallel to the first center axis, and at a second end portion facing the outlet is oriented substantially in a direction or a direction parallel to the second center axis.

A curvature characteristic does not change in the curved surface or the curved flow guide, i.e. there is no inflection point, where the curvature changes sign. Preferably, the curvature or a radius of the curved flow guide is constant between the first end portion and the second end portion, so that there is provided a continuous transition between the inlet and the outlet.

Such a design of the curved flow guide can make the redirection of the flow of the fluid and the tissue even more uniform and with less resistance.

In another aspect of the present disclosure, the valve body of the suction valve may comprise a valve seat which is configured to make circumferential contact with a sealing edge of the stem in the valve closed state of the suction valve, to thereby block the flow of fluid and mucus from the inlet to the outlet. The valve seat may be inclined with respect to the axial direction and the radial direction of the suction valve or valve cylinder or stem. The valve seat may comprise a conical engagement surface, which sealingly engages with a conical engagement surface of the stem in the valve closed state.

In other words, the valve seat may be formed on the valve body or may be formed as a portion of the valve body. Preferably, the valve seat and the valve body are formed in one piece. Particularly preferably, the valve seat is formed integrally with the valve cylinder, which is a cylindrical part or portion of the valve body.

The sealing edge may be formed on the stem or may be formed as a portion of the stem. Preferably, the remainder of the stem and the sealing edge are formed in one piece. The valve seat surrounds or forms a passage opening between the inlet and the outlet. In the closed state of the suction valve, i.e. in the valve closed state, the sealing edge of the stem is in contact with the valve seat and the passage through the suction valve is blocked. The valve seat may be oriented both at an angle to the axial direction of the valve cylinder and at an angle to the radial direction of the valve cylinder.

By angling the valve seat in this way, the flow through the suction valve can be further improved and installation space of the suction valve in the handle can be reduced at the same time.

The valve seat of the valve body and the sealing edge of the stem may be tapered outwards at a pointed or acute angle in order to form a substantially conical sealing seat.

In other words, the sealing seat may be designed as a conical sealing seat, which does not require additional seals, such as an O-ring. The valve seat and the sealing edge may be tapered in one direction towards the button element or portion. Preferably, the taper can be about 19°.

In still other words, there may be no O-ring or rubber or silicone material provided between the valve stem and the valve seat. The sealing may be achieved purely from stem-to-valve seat contact, which are made from plastic, preferably from polypropylene as this can be sourced from sustainable biobased plastics. Omitting an O-ring from the flow path further improves the flow performance through the suction valve.

To improve the sealing performance of the plastic-to-plastic sealing, the stem and the valve seat may be conical in shape which increases the sealing force compared to a simple flat perpendicular sealing surface interface. The stem and valve seat may be tapered outwards at a 19° angle relative to the longitudinal axis of the stem. The angle need not be 19° exactly.

Such a design can further reduce the number of components and the assembly effort and thus the cost of the suction valve, since the additional sealing element can be omitted.

In another aspect, the stem may comprise a sealing plate, the sealing plate may be formed at an end portion of the stem away from the button portion or element. The sealing plate may have the sealing edge on an outer edge surrounding the sealing plate i.e. a peripheral or circumferential edge.

In other words, the sealing plate may form an end portion of the stem. The sealing plate may include the sealing edge. In the closed state of the suction valve, i.e. in the valve closed state, the sealing plate can completely close the passage in the valve seat, taking up little installation space. Furthermore, the plate-shaped geometry of the sealing plate allows the longest possible travel of the stem and thus a large opening cross-section.

In another aspect, the stem may comprise a retention hook, provided at the sealing plate of the stem in a direction facing away from the button portion or element of the stem.

The retention hook or at least a portion of the retention hook may extend in a radially outward direction beyond the sealing plate.

The retention hook may be an integral portion formed in one piece with the stem.

In other words, the stem may include the retention hook, which is formed as a barb. The retention hook may be resiliently formed on the sealing plate so that the stem can be easily pushed through the valve seat in an assembly direction. In a disassembly direction directly opposite to the assembly direction, the retention hook hooks onto the valve seat and prevents the stem from being pulled out of the valve body. The retention hook may preferably be V-shaped.

In another aspect, the valve seat of the valve body may be made of an elastic material or is elastically mounted. The elastic valve seat allows the stem to be mounted into the valve body in a push-through manner.

In other words, the valve seat may be formed as a resilient member that is elastically deformable in a radial outward direction to allow an object with a slightly larger diameter than the diameter of the valve seat to pass through the valve seat, and the valve seat resiliently returns to its original shape after the object has passed through.

Such a configuration of the valve seat allows the suction valve to be assembled in a simple manner. Specifically, the suction valve may be assembled by inserting or sliding the stem into the valve body, which significantly reduces the assembly effort of the suction valve and thus the costs of the suction valve. Further, the valve body and stem may be at least largely formed in one piece, which reduces manufacturing costs and assembly costs. Similarly, the retention hook may be formed as an elastically resilient part, which can be compressed during insertion into the valve cylinder during assembly, and "spring" out i.e. decompress after passing the valve seat.

In another aspect, the suction valve may comprise a valve spring, the valve spring may be provided between the valve body and the stem, in order to force and to hold the suction valve in the closed state. The valve spring may have at least one bypass hole, which, in the state when the suction valve is closed, i.e. in the valve closed state, allows ambient air to be drawn through the valve. The valve spring may be a part separate from the stem or the valve body. Alternatively, the spring may be a preferably elastic integral part of either the valve body or the stem. It is added that the spring must not be necessarily provided. In particular, the stem can be manually moved between at least the first position and the second position without being preloaded by a spring towards one of the positions.

The valve spring may radially surround at least one area or portion between the button element or portion and the valve body. However, embodiments are also conceivable in which the valve spring is formed in the button and/ or in the stem and/ or in a surrounding sleeve. The valve spring can be made of an elastic material, preferably rubber. The spring may include at least the one, preferably two bypass holes.

By forming the bypass holes, damage to the vacuum device and/ or the endoscope can be prevented in the valve closed state. The rubber valve spring makes the suction valve easy to install, since the valve spring can be mounted on the stem and the valve body without tools. Furthermore, the valve spring in such a design can protect the suction valve in such a way that contamination is prevented from entering a gap between the stem and the valve body.

Preferably, the valve spring may be installed in the suction valve in such a way that the valve spring is preloaded.

**In** other words, the valve spring may be installed in the suction valve in a slightly compressed state, so that in a state in which no activation force acts on the suction valve from the outside, e.g. from the operator, the valve spring presses the sealing edge of the stem into the valve seat with the pretensioning force and thus increases the tightness of the suction valve.

**In** another aspect, the valve spring may be configured to form a bellows shape in a compressed state of the valve spring, thereby closing the bypass hole(s).

**In** other words, the valve spring may form at least one fold in a compressed state of the spring so that the bypass hole is closed by the spring itself.

By configuring the spring in this way, the number of components for the suction valve can be further reduced.

**In** another aspect, the stem may comprise a stop, which may be configured to be brought into contact with the valve body and prevent overpressing of the suction valve.

**In** other words, the stem may comprise the stop, preferably in the form of at least one rib, which may extend in a radial direction of the stem. **If** the button is pressed too hard, the stop comes into contact with the valve body, so that the stem cannot be pressed further into the valve body. Further, the stop may ensure that the stem and the flow guide are positioned in a pre-configured position for optimum flow in the suction valve.

Due to the integral design of the stop with the stem, the number of components of the suction valve can be further reduced, which further reduces the assembly effort and thus the assembly costs of the suction valve.

**In** another aspect, the stem may comprise a recess, the recess extending, at least in sections, normal or perpendicular to the axial direction of the stem, wherein the recess, in the open state of the suction valve, i.e. in the valve open state, connects the input and the output to each other and wherein the recess comprises the curved flow guide of the stem.

In other words, the recess may be formed on or in the stem in order to connect the inlet to the outlet in the opened state of the suction valve. The recess may be configured in such a way that at least a part of the recess is formed perpendicular to the axial extension of the stem. The recess may preferably be formed as a through hole passing through the stem. Alternatively, the recess may be a recess located on a side of the stem. The curved flow guide may be formed in or on the recess. In other words, a surface or section of the stem bounding the recess may be formed as the curved flow guide.

With such a recess in or on the stem, the suction valve can be designed to be more compact and the volume flow through the suction valve can be increased.

In another aspect, the valve body and the stem may each be formed in one piece from a plastic material, preferably polypropylene.

In other words, the valve body and/or the stem are preferably made from a thermoplastic polymer, e.g. polypropylene (PP) especially biodegradable polypropylene, polystyrene (PS), polycarbonate (PC), acrylonitrile butadiene styrene (ABS), polyoxymethylene (POM), etc. However, also other polymer materials or other materials are conceivable.

In another aspect, the suction valve may be compatible with working channel inner diameters of 1.2 mm to 3.8 mm meaning that a sphere of diameter 3.8 mm or larger is preferably able to pass through the suction valve in its activated position, i.e. in the valve open state.

In other words, the recess in the stem may have a cross-sectional area of at least 3.8 mm.

In another aspect, the suction valve may further comprise a valve end cap, which preferably is ultrasonically welded to the valve body, which avoids gluing, which may be a labor-intensive process. In an alternative embodiment, the valve end cap may be made in one piece with the valve body. However, embodiments in which the cap is glued to the valve body are also conceivable. This is particularly conceivable if the material of the valve body is not polypropylene.

In another aspect, the valve body may be provided with a guide slot or groove for guiding a corresponding pin of the stem to ensure a straight up and down or axial movement of the stem in the valve body or the valve cylinder of the valve body when actuating the suction valve.

In another aspect, the valve spring may feature preferably small bumps for sealing against the valve body.

Furthermore, in another aspect, the valve spring also may act as the movement stop for the stem displacement i.e. when the spring is fully compressed it may act as a stop and may feature bump stops.

In another aspect, the inlet and the outlet may be arranged at an angle smaller than 180° to each other in a top view, that is, in a viewing direction that corresponds to the axial direction of the valve body and the stem.

The present disclosure further relates to a method of assembling a suction valve of an endoscope as described above, the suction valve comprising a valve body, a stem and a valve spring, the valve body having an elastic valve seat, the method comprising the steps of:
- Attaching the valve spring to the stem;
- pushing the stem into the valve body;
- elastic bending of the valve seat of the valve body with the stem; and
- positioning the valve spring on the valve body.

In other words, the object of the present disclosure is also solved by the method of assembling the suction valve. Preferably, the endoscope is a single-use endoscope.

In the first step, the valve spring is attached to the stem. The valve spring may be attached to the stem, for example, in such a way that a clamping edge of the valve spring is elastically pulled on and the clamping edge is inserted into a receiving groove formed on the stem.

In the second step, the stem may be inserted into the valve body through an opening in the valve body. The insertion takes place in an actuation direction of the stem, that is, in a direction in which the stem is movable within the valve body of the suction valve.

In the third step, the stem elastically bends the valve seat, which is located inside the valve body, preferably radially outward. In other words, the stem widens the valve seat to such an extent that the stem or a section of the stem, preferably the section of the stem that has the sealing edge, can be pushed through or passes the valve seat. When the section of stem has passed the valve seat, the valve seat snaps back into its predetermined shape.

In the fourth step, the valve spring or a section of the valve spring is pulled over the valve body so that the valve spring is fixed to the valve body. For this purpose, the valve spring can be elastically expanded and then shrink back on the valve body.

Such a method allows the suction valve to be assembled without any tools, which reduces the assembly effort and thus the manufacturing costs of the suction valve.

In one aspect, the stem may comprise a retention hook and the method may further comprise the step of:
- passing a retention hook of the stem through the valve seat;
after the step of pushing the stem into the valve body.

In other words, the stem may further comprise the retention hook, which is preferably formed on a portion of the stem that is first inserted into the valve body. The retention hook is elastically mounted on the stem and acts as a barb that prevents the stem from being pulled out of the valve body once the retention hook has passed the valve seat. The retention hook ensures that the suction valve does not disassemble itself even in the event of incorrect operation or rough handling.

In still other words, the valve may be assembled by attaching the valve spring to the stem and pushing the stem into the valve body until a bottom of the stem including the retention hook is past the valve seat. The valve seat is somewhat flexible and flexes when the stem is pushed through. The retention hook is compressed as the stem is pushed through the valve body and through the valve seat after which it then expands creating a backup hard stop preventing the stem from being pulled back past the valve seat. The engagement of the sealing edge of the stem and the valve seat in itself prevent the stem from being pulled past the valve seat in a first step, before the retention hook takes effect. The valve spring is positioned on the valve body.

In another aspect, the stem may comprise a guide pin and the valve body may comprise a guide slot or groove and the method may comprise a step of
- inserting the guide pin of the stem in the guide slot;
preferably directly before, or at the same time as the step of pushing the stem into the valve body.
The present disclosure further relates to a system comprising the endoscope according to one of the above aspects and a video processing apparatus (VPA) couplable to the endoscope and capable of outputting a live image recorded by an image sensor of the endoscope on a display. The video processing apparatus may comprise a built-in display and/ or a detachable display and/or may be couplable to an external display.. The video processing apparatus may be a video processing apparatus showing an image or a video captured by an image capturing means arranged at the distal tip unit.
Furthermore, the present disclosure relates to a system, that may also be claimed independently, comprising the endoscope described before wherein the system further comprises a suction device that is connectable directly or indirectly, for example through a hose, to the outlet of the valve body of the suction valve.
The suction device may be a vacuum pump or the like. There are also embodiments imaginable, in which the suction device is directly coupled to the endoscope or is part of the endoscope.

### Brief description of the figures

The disclosure is explained in more detail below using preferred embodiments and referring to the accompanying figures.
Fig. 1 shows a side view of an endoscope according to the present disclosure, a video processing apparatus and a suction device.
Fig. 2 shows a perspective view of a half shell of the endoscope handle having a suction valve.
Fig. 3 shows the suction valve mounted in the half shell in an enlarged side view.
Fig. 4 shows the suction valve in a closed state in a sectional view.
Fig. 5 shows the suction valve in an open state in a sectional view.
Fig. 6 shows the suction valve in the closed state in a sectional perspective view.
Fig. 7 shows a valve seat of the suction valve in the closed state in an enlarged view.

The figures are schematic in nature and serve only to understand the disclosure. The features of the different embodiments can be interchanged among each other.

### Detailed description of the figures

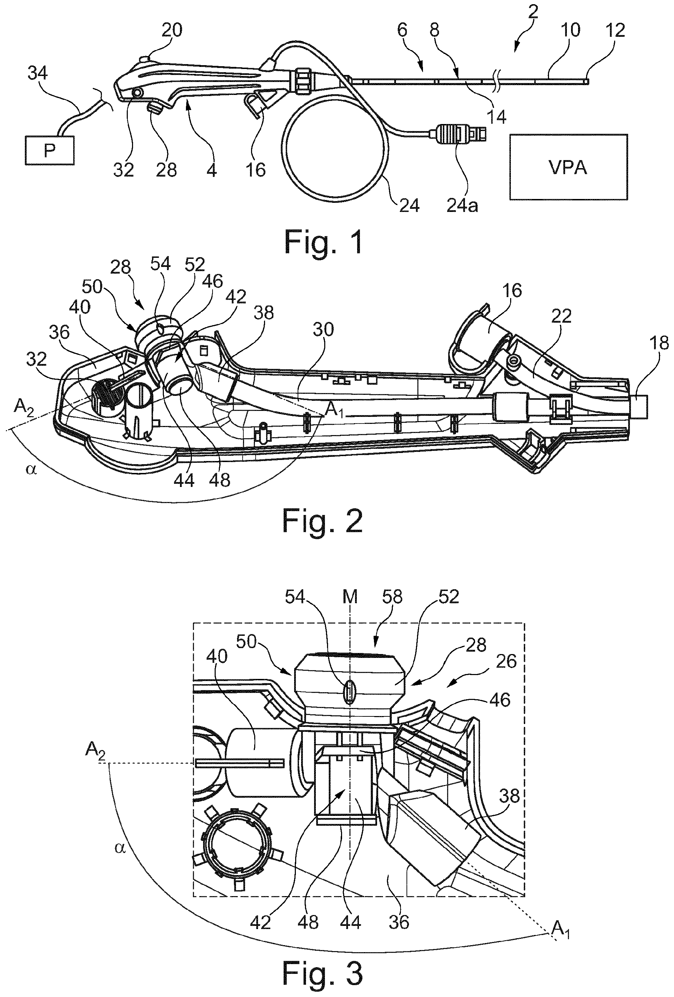

Fig. 1 shows a side view of an endoscope 2 according to the present disclosure, which is preferably a single-use endoscope. The endoscope 2 comprises: a proximal endoscope handle 4 and an insertion cord 6 extending distally from the endoscope handle 4. The insertion cord 6 is configured to be inserted into a patient's body cavity and comprises an insertion tube 8, a bending section 10 and a distal tip unit 12. The endoscope 2 further comprises a working channel 14 which extends from a biopsy port or a working channel access port 16 provided at the endoscope handle 4 to the distal tip unit 12. The working channel 14 comprises a working channel tube 18 (shown in Fig. 2), which is arranged inside the endoscope handle 4, the insertion tube 8, and the bending section 10. The endoscope handle 4 comprises an operating unit 20, formed as a lever, for bending the bending section 10 of the insertion cord 6. The working channel tube 18 and the working channel access port 16 are connected via a Y-connector 22. The endoscope 2 is connectable to a video processing apparatus VPA (schematically illustrated) via a connecting cable 24 comprising an electrical connector 24a insertable in a socket (not shown) of the video processing apparatus VPA. Alternatively the endoscope 2 may be wirelessly connectable to the video processing apparatus VPA, e.g. by a wireless HDMI connection.

When a surgical instrument or tool is inserted into the working channel 14, the surgical instrument or tool is inserted into the working channel access port 16 and guided through the Y-connector 22 into the working channel tube 18.

The endoscope 2 further comprises a suction valve 26, having a suction button 28 formed on the handle 4 and configured to control a suction power through the working channel 14 of the endoscope 2. The suction valve 26 is connected to a suction channel 30 and a vacuum port 32, which protrudes from or is embedded in the handle 4 (shown in Fig. 2). The endoscope 2 is connectable to a suction pump P (schematically illustrated) via a hose 34, which is connectable to the vacuum port 32 of the endoscope 2.

Fig. 2 shows the handle 4 in an open state. Specifically, the handle 4 is formed essentially from two half-shells 36 that form the housing of the handle and in Fig. 2, one of the two half-shells 36 is removed and all other elements of the endoscope 2, which are not necessary for an understanding of the present disclosure, are also not shown in Fig. 2 for the sake of clarity and comprehensibility of the disclosure. It is to be understood that all other mechanical, optical and electronic components, which are formed in the handle 4 in a conventional endoscope, are or may be formed in the endoscope 2 of the present disclosure.

The suction channel 30 connects the suction valve 26, which is mounted between the two half shells 36 at a proximal end portion of the handle 4, to the Y-connector 22 and runs in a longitudinal direction of the handle 4, that is, essentially in a direction, which continues the insertion cord 6. The suction valve 26 comprises an inlet 38, which is configured to connect to the suction channel 30. In other words, the inlet 38 is configured as a connector, which connects to the suction channel 30. The suction channel 30 has a tubular shape and may be in the form of an elastomeric tube or a rigid pipe. The suction valve 26 further comprises an outlet 40, which is connectable to the vacuum port 32. In other words, the outlet 40 is configured as a connector, which connects to the vacuum port 32.

In the following, the external structure of the suction valve 26 is explained in more detail with reference to Figs. 2 and 3. Fig. 3 is an enlarged view of the suction valve 26 in the assembled state, whereby the suction channel 30 and the vacuum port 32 are not shown in Fig. 3.

The suction valve 26 comprises a valve body 42, which comprises a valve cylinder 44 extending in an axial direction of the suction valve 26. The valve body 42 further comprises the inlet 38 and the outlet 40. The inlet 38 and outlet 40 each have a substantially tubular geometry with a substantially circular cross-sectional configuration. Seen in the side view of the endoscope handle in an assembled state of the suction valve in Fig. 3, the inlet 38 has a first center axis A1 and the outlet 40 has a second center axis A2. The first center axis A1 extends in a center position of the inlet 38 in a direction of extension of the inlet 38. The second center axis A2 extends in a center position of the outlet 40 in a direction of extension of the outlet 40. A first angle α is enclosed between the first center axis A1 and the second center axis A2. The first angle α is greater than 90° and less than 180°. Preferably, the angle is greater than 110°. Particularly preferably, the angle is about 120°.

The valve body 42 is formed in one piece from a plastic material, preferably polypropylene. Further the valve body 42 comprises fixing projections 46 formed thereon, which are configured to fix the suction valve 26 in the handle 4 between the half-shells 36.

The suction valve 26 further includes an end cap 48 formed on an end portion of the valve cylinder 44 facing an interior of the handle 4, and closing the valve cylinder 44. Specifically, the end cap 48 closes a substantially circular bottom opening of the valve body 42. The end cap 48 and the valve body 42 are welded together. Alternatively, it is conceivable that the end cap 48 may be fixed in place by adhesive bonding or by a latching action.

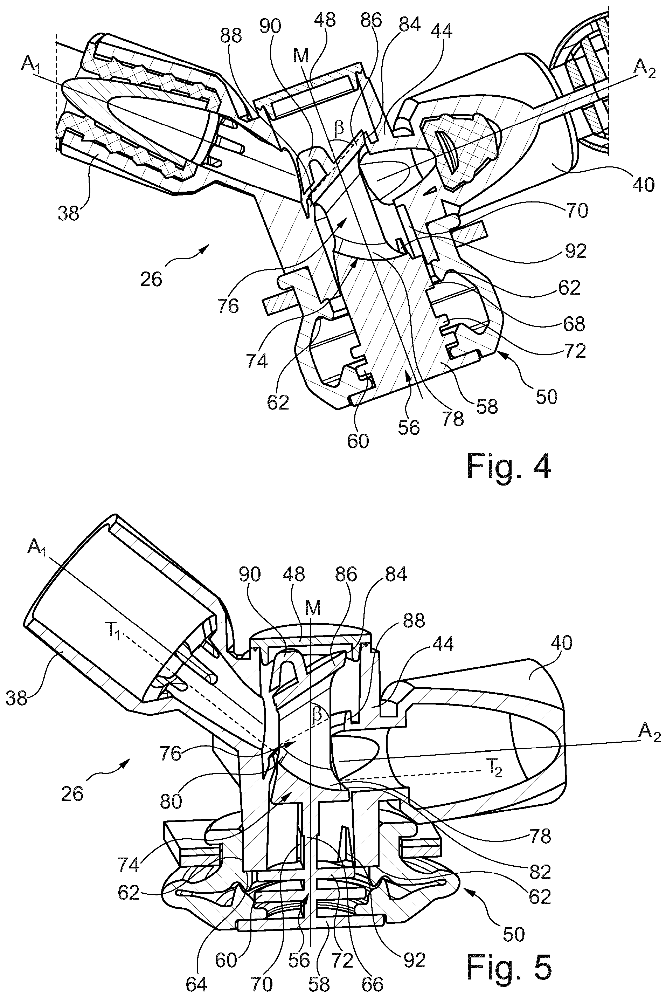

A valve spring 50 is formed on an end portion of the valve cylinder 44 opposite the end cap 48. The valve spring 50 protrudes for the most part from the handle 4 and ensures that the suction valve 26 is maintained in an initial valve closed state or is returned to the initial valve closed state without a user actuation. The specific operation of the valve spring 50 will be explained in more detail below with reference to Figs. 4 and 5. A bypass hole 54 is formed in a side surface 52 of the valve spring 50. The bypass hole 54 allows ambient air to be drawn through the suction valve 26 into the outlet 40 in a closed state of the suction valve, that is, in a state in which the volume flow from inlet 38 to outlet 40 is blocked.

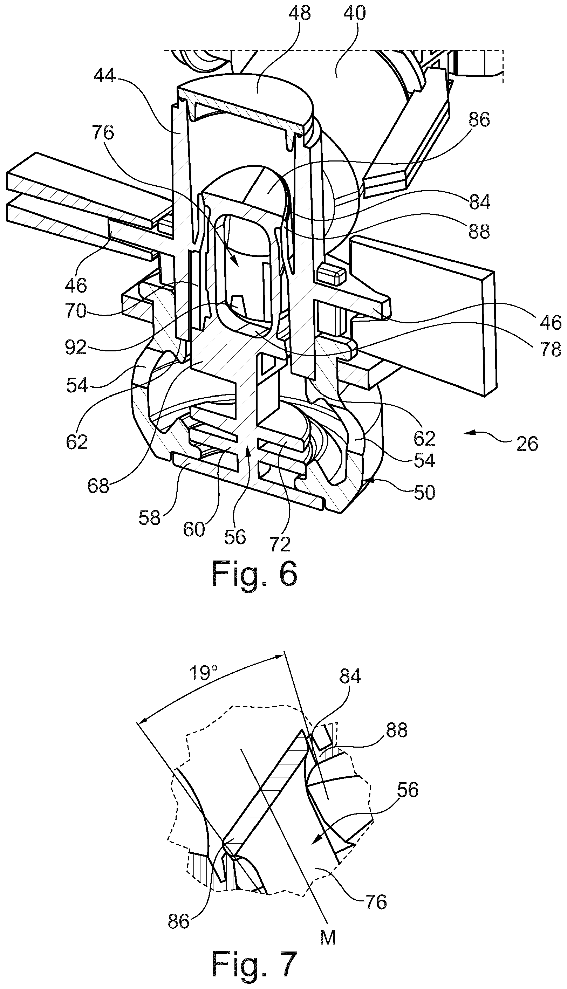

Fig. 4 shows the suction valve 26 in a sectional view in a state in which the suction valve 26 is closed. Fig. 5 shows the suction valve 26 in a sectional view in a state in which the suction valve 26 is open. Fig. 6 shows the suction valve 26 in a sectional view in a state, in which the suction valve 26 is closed. The internal structure of the suction valve 26 is explained below with reference to Figs. 4, 5 and 6.

A stem 56 is arranged in the valve cylinder 44 of the suction valve 26. The stem 56 is configured to be axially displaceable or movable in the valve cylinder 44 along an axial direction or a center axis M of the valve cylinder 44, thereby opening and closing the suction valve 26. Specifically, the stem 56 includes a button portion 58. The button portion 58 is the portion of the stem 56 that protrudes furthest from the housing of the endoscope handle 4 and forms a planar pushing portion oriented normal to the longitudinal extension or axial direction of the stem 56 and is configured to be pushed by an operator to move the stem 56 along the axial direction or center axis M in the valve cylinder 44. The button portion 58 is surrounded by the valve spring 50 in a circumferential direction. The button portion 58 and the valve spring 50 form the suction button 28 protruding from the handle 4.

A detent portion 60 is formed adjacent to the button portion 58. The button portion 58 and the detent portion 60 are formed to receive a clamping edge 62 of the valve spring 50 between them. The valve spring 50 is made of an elastomeric material and therefore clamps between the button portion 58 and the detent portion 60. The detent portion 60 is substantially rib-shaped and extends, parallel to the button portion 58, in a direction radially outward with respect to the axial direction of the stem 56. At an end of the valve spring 50 facing away from the button portion 58, that is a connection section 62 of the valve spring 50 facing the valve cylinder 44, the valve spring 50 radially surrounds the valve cylinder 44. Here, the valve spring 50 clamps tightly onto the valve cylinder 44 due to its elastic properties. The valve spring 50 is formed with a circumferential sealing protrusion 64 that circumferentially contacts and seals an opening edge of the valve cylinder 44.

The stem 56 comprises a shaft portion 66 that extends from the button portion 58 into the valve cylinder 44. The shaft portion 66 has a substantially plate-like shape.

Guide pins 68 are formed in a width direction of the shaft portion 66, that slide in grooves 70 formed in an inner wall of the valve cylinder 44 to prevent rotation of the stem 56 relative to the valve cylinder 44. Furthermore, a stop 72 is formed on the shaft portion 66. The stop 72 is oriented parallel to the button portion 58 and the detent portion 60 and prevents overpressing of the stem 56 relative to the valve body 42. The stop 72, like the detent portion 60, is formed as a type of rib. Here, overpressing means that a portion of the stem 56 facing away from the button portion 58 is pressed against or comes into contact with the end cap 48.

Further, the stem 56 includes a piston portion 74. The piston portion 74 is formed at an end of the shaft portion 66 opposed to the button portion 58. The piston portion 74 comprises a through hole 76. The through hole 76 penetrates the piston portion 74 substantially in a direction normal or perpendicular to the axial direction of the stem 56. The through hole 76 is provided and configured to connect the inlet 38 and the outlet 40 of the suction valve 26 in an activated state of the suction valve 26 (shown in Fig. 5).

The through hole 76 comprises a curved flow guide 78 on or as a wall portion, specifically on the wall portion or wall surface oriented toward the button portion 58. The curved flow guide 78 is configured to redirect the flow from the inlet 38 to the outlet 40 as uniformly as possible. The curved flow guide 78 has no edges within a surface of the curved flow guide 78 and a substantially uniform curvature or a uniformly changing curvature. Fig. 5 shows in particular that in the valve open state the inlet 38, the outlet 40 and the curved flow guide 78 of the stem 56 are arranged and adapted to each other so as to provide a curved sideways flow path for the fluid and the mucus through the suction valve 26.

At a first edge 80 of the curved flow guide 78 facing the inlet 38, the curvature of the curved flow guide 78 is such that a first tangent T1 of the curved flow guide 78 is oriented parallel to the first center axis A1. At a second edge 82 of the curved flow guide 78 facing the outlet 40, the curvature of the curved flow guide 78 is such that a second tangent T2 of the curved flow guide 78 is oriented parallel to the second center axis A2.

The piston portion 74 further includes a circumferential sealing edge 84 formed on a sealing plate 86 that forms an end portion of the stem 56 away from the button portion 58 and bounds the through hole 76. The sealing plate 86 is configured to shut off the flow through the suction valve 26. Specifically, the sealing plate 86 is configured to be seated in a valve seat 88 in the closed state of the suction valve 26, with the sealing edge 84 sealing against the valve seat 88.

The valve seat 88 is formed in the valve cylinder 44. In particular, the valve seat 88 is formed integrally with the valve cylinder 44 from the same material. The valve seat 88 and thus also the sealing plate 86 with the sealing edge 84 are oriented inclined to the axial direction or center axis M. A second angle β, which is formed between the valve seat 88 and the center axis M, is smaller than 90°. The valve seat 88 is elastic. This means that the valve seat 88 can be expanded in an elastic manner and then elastically returns to its predetermined shape. The valve seat 88 and the sealing plate 86, having the sealing edge 84, are formed from the same material. In other words, the sealing between the valve seat 88 and the sealing edge 84 is a seal between identical materials, in particular plastics, preferably polypropylene. The sealing plate 86 is pressed into the valve seat 88 with a preloaded force. In other words, the valve spring 50 is preloaded in the closed state of the suction valve 26 shown in Fig. 4, so that the valve spring 50 presses the sealing plate 86 with the sealing edge 84 into the valve seat 88 with the preload force and keeps the suction valve 26 closed in the closed state, that is, in a state in which the suction valve 26 is not operated by the operator.

A retention hook 90 is formed on a surface of the sealing plate 86 facing away from the through hole 76. The retention hook 90 is formed as a resilient barb, which prevents the stem 56 from being pulled out from the valve cylinder 44. Specifically, the retention hook 90 latches onto the valve seat 88 when the sealing edge 84 of the sealing plate 86 overcomes the valve seat 88.

Fig. 7 shows an enlarged view of the sealing plate 86 in the valve seat 88. Both the valve seat 88 and the sealing edge 84 taper in a direction towards the through hole 76. In other words, the valve seat 88 and the sealing edge 84 are a conical valve seat 88 and a conical sealing edge 84, which are inclined at a third angle, the third angle being preferably 19°, to increase the sealing effect of the suction valve 26. 19° are to be understood here as an example. Of course, other angles are also possible.

In the following, the function of the suction valve 26 is briefly described with reference to the figures.

The suction pump P is connected to the vacuum port 32 via the hose 34 and generates a vacuum/ suction force at the outlet 40. The suction valve 26 is in the closed state shown in Fig. 4. In other words, the sealing plate 86 with the sealing edge 84 is seated in the valve seat 88 of the valve cylinder 44 by the preload force applied by the valve spring 50. In the closed state of the suction valve 26, the suction pump P draws in air from the environment. This air is sucked into the suction valve 26 from the environment through the bypass holes 54 in the valve spring 50 and reaches the outlet 40 via bypass channels 92, which are formed in the inner wall of the valve cylinder 44. This effectively prevents the suction pump P from working against a big resistance in a closed state of the suction valve 26, which could damage the suction pump P and/ or the endoscope 2.

Now, when the operator starts the suction function of the endoscope 2, that is, when the button portion 58 of the stem 56 is pressed hard enough by a finger of the operator to overcome the spring force of the valve spring 50, the stem 56 slides or moves in the valve cylinder 44. The stem 56 is guided in the grooves 70 via the pins 68. In this process, the sealing plate 86 with the sealing edge 84 leaves the valve seat 88 and opens the through hole 76. Specifically, the through hole 76 is axially displaced with the stem 56 in the valve cylinder 44 such that the through hole 76 now connects the outlet 40 to the inlet 38 of the suction valve 26. The suction force of the suction pump P can be transmitted to the working channel 14 via the suction channel 30 and fluid and mucus of the patient can be sucked off or aspirated. In the suction valve 26, the fluid and mucus is only deflected once through the curved flow guide 78, so that clogging of the suction valve 26 can be prevented. The fluid and mucus are diverted as evenly and gently as possible through the curved flow guide 78. The defined angle between inlet 38 and outlet 40 can further reduce the risk of clogging and enable gentle diversion through the curved flow guide 78.

Pressing the button portion 58 folds the valve spring 50 like a bellows (see Fig. 5). Two inner sections of the valve spring 50 come into contact in such a way that they lie sealingly on top of each other and close off the bypass holes 54. The stop 72 gives the operator a secure feeling that the suction valve 26 is fully open. In addition, the stop 72 prevents overpressing, so that the operator can press the button portion 58 fully without risk and without thinking.

When the operator wants to interrupt or stop the suction, he simply releases the button portion 58 and the spring force of the valve spring 50 moves the stem 56 axially through the valve cylinder 44 until the sealing plate 86 with the sealing edge 84 is seated again in the valve seat 88 and the suction valve 26 is closed. This automatically stops the elastic valve spring 50 from folding and opens the bypass holes 54 so that the suction pump P sucks in ambient air.

### List of reference numbers

- 2: endoscope
- 4: endoscope handle
- 6: insertion cord
- 8: insertion tube
- 10: bending section
- 12: distal tip unit
- 14: working channel
- 16: working channel access port
- 18: working channel tube
- 20: operating unit
- 22: Y-connector
- 24: connecting cable
- 24a: electrical connector
- 26: suction valve
- 28: suction button
- 30: suction channel
- 32: vacuum port
- 34: hose
- 36: half-shell
- 38: inlet
- 40: outlet
- 42: valve body
- 44: valve cylinder
- 46: fixing projections
- 48: end cap
- 50: valve spring
- 52: side surface
- 54: bypass hole
- 56: stem
- 58: button portion
- 60: detent portion
- 62: clamping edge
- 64: sealing protrusion
- 66: shaft portion
- 68: guide pin
- 70: groove/ slot
- 72: stop
- 74: piston portion
- 76: through hole
- 78: curved flow guide
- 80: first edge
- 82: second edge
- 84: sealing edge
- 86: sealing plate
- 88: valve seat
- 90: retention hook
- 92: bypass channel
- T1: first tangent
- T2: second tangent
- M: center axis of the valve cylinder
- α: first angle
- β: second angle
- A1: first center axis
- A2: second center axis
- P: suction pump
- VPA: video processing apparatus

## Claims

1. An endoscope (2) comprising:
a proximal endoscope handle (4) or interface;
an insertion cord (6) extending from the endoscope handle (4) or interface and configured to be inserted into a patient's body cavity;
a working channel (14) configured to let fluid and/or mucus sucked from the patient's body cavity flow therethrough;
the endoscope handle (4) or interface comprising:
a suction valve (26) configured to control a suction through the working channel (14) and having a valve closed state and a valve open state;
the suction valve (26) comprising:
a valve body (42) having: a valve cylinder (44); an inlet (38) connected to the working channel (14); and an outlet (40) provided and configured to be connected to a suction device (P); and
a stem (56) movable axially in the valve cylinder (44) between at least a first position, in which the suction valve (26) is in the valve closed state, and a second position, in which the suction valve (26) is in the valve open state; wherein
the inlet (38) is connected to and extends away from an outer cylindrical surface of the valve cylinder (44),
the outlet (40) is connected to and extends away from the outer cylindrical surface of the valve cylinder (44),
the stem (56) comprises a curved flow guide (78) configured to smoothly guide the fluid and/or mucus from the inlet (38) to the outlet (40) in the valve open state, wherein the curved flow guide (78) is designed such that it provides a curved, continuous transition between the inlet (38) and the outlet (40) in the valve open state, and
the inlet (38), the outlet (40) and the curved flow guide (78) are arranged and adapted to each other so as to provide a curved sideways flow path for the fluid and/or mucus through the suction valve (26) in the valve open state, wherein
the curved sideways flow path is provided by the curved flow guide (78) of the stem (56) in combination with an enclosed angle (α) between a first orientation or center axis (A1) of the inlet (38) and a second orientation or center axis (A2) of the outlet (40) being larger than 100° and smaller than 180° seen in a side view of the endoscope handle (4) or interface in an assembled state of the suction valve (26) into the endoscope handle (4) or interface,
a curvature of the curved flow guide (78) is configured such that the flow guide (78) at a first end portion facing the inlet (38) is oriented substantially in the direction or a direction parallel to the first center axis (A1), and at a second end portion facing the outlet (40) is oriented substantially in a direction or a direction parallel to the second center axis (A2), and
a curvature characteristic does not change in the curved flow guide (78), i.e. there is no inflection point, where the curvature changes sign.

2. The endoscope (2) according to claim 1, wherein the valve body (42) of the suction valve (26) comprises a valve seat (88) which is configured to make circumferential contact with a sealing edge (84) of the stem (56) in the valve closed state of the suction valve (26), thereby blocking a flow of fluid and mucus from the inlet (38) to the outlet (40).

3. The endoscope (2) according to claim 2, wherein the valve seat (88) of the valve body (42) and the sealing edge (84) of the stem (56) are tapered at an acute angle in order to form a substantially conical sealing seat.

4. The endoscope (2) according to claim 3, wherein the stem (56) comprises a sealing plate (86), the sealing plate (86) being formed at an end portion of the stem (56), the sealing plate (86) having the sealing edge (84) on an outer edge surrounding the sealing plate (86).

5. The endoscope (2) according to any one of claims 2 to 4, wherein the valve seat (88) of the valve body (42) is made of an elastic material or is elastically mounted therefore allowing the stem (56) to be assembled into the valve body (42) in a push-through manner.

6. The endoscope (2) according to claim 5, wherein the stem (56) comprises a retention hook (90), which when the stem (56) is assembled into the valve body (42) in an assembly direction can be pushed through the valve seat (88), and which when the stem (56) is pulled in a disassembly direction opposite to the assembly direction hooks onto the valve seat (88), thereby preventing the stem (56) from being pulled out of the valve body (42).

7. The endoscope (2) according to any one of claims 1 to 6, wherein the suction valve (26) comprises a valve spring (50), the valve spring (50) being formed between the valve body (42) and the stem (56), the valve spring (50) being configured to push the stem (56) to the first position, in which the suction valve (26) is in the valve closed state, wherein the valve spring (50) has at least one bypass hole (54), which in the valve closed state allows ambient air to be drawn through the suction valve (26).

8. The endoscope (2) according to claim 7, wherein the valve spring (50) is configured to form a bellows shape in a compressed state of the valve spring (50), thereby closing the bypass hole (54).

9. The endoscope (2) according to any one of claims 1 to 8, wherein the stem (56) comprises a stop (72), which is configured to be brought into contact with the valve body (42) and to prevent overpressing of the stem (56).

10. The endoscope (2) according to any one of claims 1 to 9, wherein the stem (56) comprises a recess (76), the recess (76) extending, at least in sections, perpendicular to an axial direction of the stem (56), wherein the recess (76) in the valve open state connects the inlet (38) and the outlet (40) to each other and wherein the recess (76) comprises the curved flow guide (78).

11. The endoscope (2) according to any one of claims 1 to 10, wherein the valve body (42) and the stem (56) are each formed in one piece from a thermoplastic material, preferably polypropylene.

12. A method of assembling an endoscope (2) according to claim 1 in combination with claims 5 and 7, the suction valve (26) comprising the valve body (42), the stem (56) and the valve spring (50), the valve body (42) having the elastic valve seat (88), the method comprising the steps of:
- Attaching the valve spring (50) to the stem (56);
- Pushing the stem (56) into the valve body (42);
- Elastically bending the valve seat (88) of the valve body (42) with the stem (56); and
- Positioning the valve spring (50) on the valve body (42).

13. The method according to claim 12 in combination with claim 6, wherein the stem (56) comprises the retention hook (90) and the method further comprises the step of
- Passing the retention hook (90) of the stem (56) through the valve seat (88);
after the step of pushing the stem (56) into the valve body (42).

14. A system comprising an endoscope (2) according to one of claims 1 to 11 and a video processing apparatus (VPA).

## Patentansprüche

1. Endoskop (2), umfassend:
einen proximalen Endoskopgriff (4) oder eine Schnittstelle;
einen Einführstrang (6), der sich vom Endoskopgriff (4) oder der Schnittstelle erstreckt und so ausgebildet ist, dass er in eine Körperhöhle eines Patienten eingeführt werden kann;
einen Arbeitskanal (14), der so ausgebildet ist, dass aus der Körperhöhle des Patienten abgesaugte Flüssigkeit und/oder Schleim durch ihn hindurchströmen können;
wobei der Endoskopgriff (4) oder die Schnittstelle umfasst:
ein Saugventil (26), das so ausgebildet ist, dass es ein Saugen durch den Arbeitskanal (14) steuert, und das einen geschlossenen Ventilzustand und einen offenen Ventilzustand aufweist;
wobei das Saugventil (26) umfasst:
einen Ventilkörper (42) mit: einem Ventilzylinder (44); einem mit dem Arbeitskanal (14) verbundenen Einlass (38); und einem Auslass (40), der so ausgebildet und konfiguriert ist, dass er mit einer Ansaugvorrichtung (P) verbunden werden kann; sowie
einen Schaft (56), der im Ventilzylinder (44) axial zwischen mindestens einer ersten Position, in der sich das Saugventil (26) im geschlossenen Zustand befindet, und einer zweiten Position, in der sich das Saugventil (26) im geöffneten Zustand befindet, bewegbar ist; wobei
der Einlass (38) mit einer äußeren zylindrischen Oberfläche des Ventilzylinders (44) verbunden ist und von dieser wegführt,
der Auslass (40) mit der äußeren zylindrischen Oberfläche des Ventilzylinders (44) verbunden ist und von dieser wegführt,
der Schaft (56) eine gekrümmte Strömungsführung (78) umfasst, die so ausgebildet ist, dass sie das Fluid und/oder den Schleim vom Einlass (38) zum Auslass (40) im geöffneten Zustand des Ventils sanft leitet, wobei die gekrümmte Strömungsführung (78) so ausgelegt ist, dass sie im geöffneten Zustand des Ventils einen gekrümmten, kontinuierlichen Übergang zwischen dem Einlass (38) und dem Auslass (40) bildet, und
der Einlass (38), der Auslass (40) und die gekrümmte Strömungsführung (78) so zueinander angeordnet und aufeinander abgestimmt sind, dass sie im geöffneten Zustand des Ventils einen gekrümmten, seitlichen Strömungsweg für das Fluid und/oder den Schleim durch das Absaugventil (26) bilden, wobei
der gekrümmte, seitliche Strömungsweg gebildet wird durch die gekrümmte Strömungsführung (78) des Schafts (56) in Verbindung mit einem eingeschlossenen Winkel (α) zwischen einer ersten Ausrichtung oder Mittelachse (A1) des Einlasses (38) und einer zweiten Ausrichtung oder Mittelachse (A2) des Auslasses (40) größer als 100° und kleiner als 180° gesehen in einer Seitenansicht des Endoskopgriffs (4) oder der Schnittstelle in dem eingebauten Zustand des Saugventils (26) in den Endoskopgriff (4) oder die Schnittstelle,
eine Krümmung der gekrümmten Strömungsführung (78) so ausgelegt ist, dass die Strömungsführung (78) an einem dem Einlass (38) zugewandten ersten Endabschnitt im Wesentlichen in Richtung der ersten Mittelachse (A1) oder in einer dazu parallelen Richtung ausgerichtet ist, und an einem dem Auslass (40) zugewandten zweiten Endabschnitt im Wesentlichen in einer Richtung der zweiten Mittelachse (A2) oder in einer dazu parallelen Richtung ausgerichtet ist, und
sich eine Krümmungscharakteristik in der gekrümmten Strömungsführung nicht ändert, d. h., es keinen Wendepunkt gibt, an dem die Krümmung ihr Vorzeichen wechselt.

2. Endoskop (2) nach Anspruch 1, wobei der Ventilkörper (42) des Saugventils (26) einen Ventilsitz (88) aufweist, der so ausgebildet ist, dass er im geschlossenen Zustand des Saugventils (26) einen Umfangskontakt mit einer Dichtungskante (84) des Schafts (56) herstellt, wodurch ein Fluss von Fluid und Schleim vom Einlass (38) zum Auslass (40) blockiert wird.

3. Endoskop (2) nach Anspruch 2, wobei der Ventilsitz (88) des Ventilkörpers (42) und die Dichtungskante (84) des Schafts (56) unter einem spitzen Winkel verjüngt sind, um einen im Wesentlichen konischen Dichtungssitz zu bilden.

4. Endoskop (2) nach Anspruch 3, wobei der Schaft (56) eine Dichtungsplatte (86) umfasst, wobei die Dichtungsplatte (86) an einem Endabschnitt des Schafts (56) ausgebildet ist und die Dichtungsplatte (86) die Dichtungskante (84) an einer die Dichtungsplatte (86) umgebenden Außenkante aufweist.

5. Endoskop (2) nach einem der Ansprüche 2 bis 4, wobei der Ventilsitz (88) des Ventilkörpers (42) aus einem elastischen Material besteht oder elastisch montiert ist, wodurch der Schaft (56) durch Durchdrücken in den Ventilkörper (42) einbaubar ist.

6. Endoskop (2) nach Anspruch 5, wobei der Schaft (56) einen Haltehaken (90) aufweist, der, wenn der Schaft (56) in einer Einbaurichtung in den Ventilkörper (42) eingebaut wird, durch den Ventilsitz (88) geschoben werden kann, und der, wenn der Schaft (56) in einer der Einbaurichtung entgegengesetzten Demontagerichtung gezogen wird, am Ventilsitz (88) einhakt und dadurch verhindert, dass der Schaft (56) aus dem Ventilkörper (42) herausgezogen wird.

7. Endoskop (2) nach einem der Ansprüche 1 bis 6, wobei das Saugventil (26) eine Ventilfeder (50) umfasst, wobei die Ventilfeder (50) zwischen dem Ventilkörper (42) und dem Schaft (56) ausgebildet ist, wobei die Ventilfeder (50) so ausgebildet ist, dass sie den Schaft (56) in die erste Position drückt, in der sich das Saugventil (26) im geschlossenen Zustand befindet, wobei die Ventilfeder (50) mindestens eine Bypass-Öffnung (54) aufweist, die im geschlossenen Zustand des Ventils das Ansaugen von Umgebungsluft durch das Saugventil (26) ermöglicht.

8. Endoskop (2) nach Anspruch 7, wobei die Ventilfeder (50) so ausgebildet ist, dass sie in einem zusammengedrückten Zustand der Ventilfeder (50) eine Balgform bildet und dadurch die Bypass-Öffnung (54) verschließt.

9. Endoskop (2) nach einem der Ansprüche 1 bis 8, wobei der Schaft (56) einen Anschlag (72) aufweist, der so ausgebildet ist, dass er mit dem Ventilkörper (42) in Kontakt gebracht werden kann und ein zu starkes Eindrücken des Schafts (56) verhindert.

10. Endoskop (2) nach einem der Ansprüche 1 bis 9, wobei der Schaft (56) eine Aussparung (76) aufweist, wobei sich die Aussparung (76) zumindest abschnittsweise senkrecht zu einer axialen Richtung des Schafts (56) erstreckt, wobei die Aussparung (76) im geöffneten Zustand des Ventils den Einlass (38) und den Auslass (40) miteinander verbindet und wobei die Aussparung (76) die gekrümmte Strömungsführung (78) umfasst.

11. Endoskop (2) nach einem der Ansprüche 1 bis 10, wobei der Ventilkörper (42) und der Schaft (56) jeweils einstückig aus einem thermoplastischen Material, vorzugsweise Polypropylen, geformt sind.

12. Verfahren zum Zusammenbau eines Endoskops (2) gemäß Anspruch 1 in Kombination mit den Ansprüchen 5 und 7, wobei das Saugventil (26) den Ventilkörper (42), den Schaft (56) und die Ventilfeder (50) umfasst, wobei der Ventilkörper (42) den elastischen Ventilsitz (88) aufweist, wobei das Verfahren die folgenden Schritte umfasst:
- Anbringen der Ventilfeder (50) an dem Schaft (56);
- Einschieben des Schafts (56) in den Ventilkörper (42);
- elastisches Biegen des Ventilsitzes (88) des Ventilkörpers (42) mit dem Schaft (56); und
- Positionieren der Ventilfeder (50) am Ventilkörper (42).

13. Verfahren nach Anspruch 12 in Kombination mit Anspruch 6, wobei der Schaft (56) den Haltehaken (90) umfasst und das Verfahren ferner den folgenden Schritt umfasst:
- Führen des Haltehakens (90) des Schafts (56) durch den Ventilsitz (88);
nach dem Schritt des Einschiebens des Schafts (56) in den Ventilkörper (42).

14. System, umfassend ein Endoskop (2) nach einem der Ansprüche 1 bis 11 und eine Videoverarbeitungsvorrichtung (VPA).

## Revendications

1. Endoscope (2) comprenant :
une poignée d'endoscope proximale (4) ou une interface ;
un cordon d'insertion (6) s'étendant de la poignée d'endoscope (4) ou de l'interface et configuré pour être inséré dans une cavité corporelle d'un patient ;
un canal de travail (14) configuré pour laisser du fluide et/ou du mucus aspiré de la cavité corporelle du patient s'écouler par celui-ci ;
la poignée d'endoscope (4) ou l'interface comprenant :
une soupape d'aspiration (26) configurée pour commander une aspiration à travers le canal de travail (14) et présentant un état fermé de soupape et un état ouvert de soupape ;
la soupape d'aspiration (26) comprenant :
un corps de soupape (42) présentant : un cylindre de soupape (44) ; une entrée (38) reliée au canal de travail (14) ; et une sortie (40) prévue et configurée pour être reliée à un dispositif d'aspiration (P) ; et
une tige (56) mobile axialement dans le cylindre de soupape (44) entre au moins une première position dans laquelle la soupape d'aspiration (26) est dans l'état fermé de soupape et une seconde position dans laquelle la soupape d'aspiration (26) est dans l'état ouvert de soupape ; dans lequel
l'entrée (38) est reliée à et s'étend à l'opposé d'une surface cylindrique extérieure du cylindre de soupape (44),
la sortie (40) est reliée à et s'étend à l'écart de la surface cylindrique extérieure du cylindre de soupape (44),
la tige (56) comprend un guide d'écoulement incurvé (78) configuré pour guider en douceur le fluide et/ou le mucus de l'entrée (38) à la sortie (40) dans l'état ouvert de soupape, dans lequel le guide d'écoulement incurvé (78) est conçu de sorte qu'il fournit une transition incurvée continue entre l'entrée (38) et la sortie (40) dans l'état ouvert de soupape, et
l'entrée (38), la sortie (40) et le guide d'écoulement incurvé (78) sont agencés et adaptés les uns aux autres de manière à fournir un chemin d'écoulement latéral incurvé pour le fluide et/ou le mucus à travers la soupape d'aspiration (26) dans l'état ouvert de soupape, dans lequel
le chemin d'écoulement latéral incurvé est fourni par le guide d'écoulement incurvé (78) de la tige (56) en combinaison avec un angle fermé (α) entre une première orientation ou axe central (A1) de l'entrée (38) et une seconde orientation ou axe central (A2) de la sortie (40) étant supérieur à 100° et inférieur à 180° vu dans une vue latérale de la poignée d'endoscope (4) ou de l'interface dans un état assemblé de la soupape d'aspiration (26) dans la poignée d'endoscope (4) ou l'interface,
la courbure du guide d'écoulement incurvé (78) est configurée de sorte que le guide d'écoulement (78), au niveau d'une première partie d'extrémité faisant face à l'entrée (38), soit orienté sensiblement dans la direction ou une direction parallèle au premier axe central (A1), et au niveau d'une seconde partie d'extrémité faisant face à la sortie (40), soit orienté sensiblement dans une direction ou une direction parallèle au second axe central (A2), et
une caractéristique de courbure ne change pas dans le guide d'écoulement incurvé (78), c'est-à-dire qu'il n'y a pas de point d'inflexion au niveau duquel la courbure change de signe.

2. Endoscope (2) selon la revendication 1, dans lequel le corps de soupape (42) de la soupape d'aspiration (26) comprend un siège de soupape (88) qui est configuré pour établir un contact circonférentiel avec un bord d'étanchéité (84) de la tige (56) à l'état fermé de soupape de la soupape d'aspiration (26), bloquant ainsi un flux de fluide et de mucus de l'entrée (38) à la sortie (40).

3. Endoscope (2) selon la revendication 2, dans lequel le siège de soupape (88) du corps de soupape (42) et le bord d'étanchéité (84) de la tige (56) sont coniques à un angle aigu afin de former un siège d'étanchéité sensiblement conique.

4. Endoscope (2) selon la revendication 3, dans lequel la tige (56) comprend une plaque d'étanchéité (86), la plaque d'étanchéité (86) étant formée au niveau d'une partie d'extrémité de la tige (56), la plaque d'étanchéité (86) présentant le bord d'étanchéité (84) sur un bord extérieur entourant la plaque d'étanchéité (86).

5. Endoscope (2) selon l'une quelconque des revendications 2 à 4, dans lequel le siège de soupape (88) du corps de soupape (42) est fait d'un matériau élastique ou est monté élastiquement permettant ainsi à la tige (56) d'être assemblée dans le corps de soupape (42) de manière à la faire passer.

6. Endoscope (2) selon la revendication 5, dans lequel la tige (56) comprend un crochet de rétention (90) qui, lorsque la tige (56) est assemblée dans le corps de soupape (42) dans une direction d'assemblage, peut être poussé à travers le siège de soupape (88), et qui, lorsque la tige (56) est tirée dans une direction de démontage opposée à la direction d'assemblage, s'accroche au siège de soupape (88), empêchant ainsi la tige (56) d'être retirée du corps de soupape (42).

7. Endoscope (2) selon l'une quelconque des revendications 1 à 6, dans lequel la soupape d'aspiration (26) comprend un ressort de soupape (50), le ressort de soupape (50) étant formé entre le corps de soupape (42) et la tige (56), le ressort de soupape (50) étant configuré pour pousser la tige (56) vers la première position dans laquelle la soupape d'aspiration (26) est dans l'état fermé de soupape, dans lequel le ressort de soupape (50) présente au moins un trou de dérivation (54) qui, dans l'état fermé de soupape, permet à l'air ambiant d'être aspiré à travers la soupape d'aspiration (26).

8. Endoscope (2) selon la revendication 7, dans lequel le ressort de soupape (50) est configuré pour former une forme de soufflet à l'état comprimé du ressort de soupape (50), fermant ainsi le trou de dérivation (54).

9. Endoscope (2) selon l'une quelconque des revendications 1 à 8, dans lequel la tige (56) comprend une butée (72) qui est configurée pour être mise en contact avec le corps de soupape (42) et pour empêcher la surpression de la tige (56).

10. Endoscope (2) selon l'une quelconque des revendications 1 à 9, dans lequel la tige (56) comprend un évidement (76), l'évidement (76) s'étendant, au moins par sections, perpendiculairement à une direction axiale de la tige (56), dans lequel l'évidement (76) dans l'état ouvert de soupape relie l'entrée (38) et la sortie (40) l'une à l'autre et dans lequel l'évidement (76) comprend le guide d'écoulement incurvé (78).

11. Endoscope (2) selon l'une quelconque des revendications 1 à 10, dans lequel le corps de soupape (42) et la tige (56) sont chacun formés d'une seule pièce à partir d'un matériau thermoplastique, de préférence du polypropylène.

12. Procédé d'assemblage d'un endoscope (2) selon la revendication 1 en combinaison avec les revendications 5 et 7, la soupape d'aspiration (26) comprenant le corps de soupape (42), la tige (56) et le ressort de soupape (50), le corps de soupape (42) présentant le siège de soupape élastique (88), le procédé comprenant les étapes suivantes :
- la fixation du ressort de soupape (50) à la tige (56) ;
- la poussée de la tige (56) dans le corps de soupape (42) ;
- la flexion élastique du siège de soupape (88) du corps de soupape (42) avec la tige (56) ; et
- le positionnement du ressort de soupape (50) sur le corps de soupape (42).

13. Procédé selon la revendication 12 en combinaison avec la revendication 6, dans lequel la tige (56) comprend le crochet de rétention (90) et le procédé comprend en outre l'étape suivante
- le passage du crochet de rétention (90) de la tige (56) à travers le siège de soupape (88) ;
après l'étape de poussée de la tige (56) dans le corps de soupape (42).

14. Système comprenant un endoscope (2) selon l'une des revendications 1 à 11 et un appareil de traitement vidéo (VPA).
